# EUROPÄISCHE PATENTSCHRIFT

(11) **EP 3 633 400 B1**
(45) Veröffentlichungstag und Bekanntmachung des Hinweises auf die Patenterteilung: **21.09.2022**
(21) Anmeldenummer: 18198141.6
(22) Anmeldetag: 02.10.2018
(51) Int. Cl.: G01R 33/36

(54) **MAGNETRESONANZTOMOGRAPH SOWIE SYSTEM UND VERFAHREN ZUM VERHINDERN VON ÜBERSPRECH-STÖRUNGEN**
MAGNETIC RESONANCE TOMOGRAPH AND SYSTEM AND METHOD FOR PREVENTING CROSS-TALK
TOMOGRAPHE À RÉSONANCE MAGNÉTIQUE AINSI QUE SYSTÈME ET PROCÉDÉ PERMETTANT D'ÉVITER DES INTERFÉRENCES DE DIAPHONIE

(43) Veröffentlichungstag der Anmeldung: 08.04.2020
(73) Patentinhaber: Siemens Healthcare GmbH, 91052 Erlangen (DE)
(72) Erfinder: Biber, Stephan, 91056 Erlangen (DE); Grodzki, David, 91058 Erlangen (DE)

(56) Entgegenhaltungen:
- DE-A1-102016 223 478
- JP-A- S60 253 436
- US-A1- 2006 279 281
- US-A1- 2011 118 588
- US-A1- 2013 053 678
- US-A1- 2016 231 403

## Beschreibung

Die Erfindung betrifft einen Magnetresonanztomographen, ein System aus zwei Magnetresonanztomographen sowie ein Verfahren zum Betrieb, die wechselseitige Störungen zwischen Magnetresonanztomographen reduzieren.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, auch als Magnetresonanzsignal bezeichnet, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Die erzeugte Darstellung gibt eine räumliche Dichteverteilung der Spins an.

Die Dämpfung zwischen Anregungspuls und dem danach vom Patienten bzw. einer Probe ausgesendeten Magnetresonanzsignale beträgt über 100 dB. Dadurch kommt es vor, dass trotz Hochfrequenzabschirmungskabinen bei in benachbarten Räumen arbeitenden Magnetresonanztomographen Anregungspulse eines Systems jeweils die gleichzeitige Erfassung eines Magnetresonanzsignals in dem benachbarten Magnetresonanztomographen stört. Üblicherweise werden derartige Störungen aufgrund des unregelmäßigen Auftretens nicht erkannt und für sporadische Störungen gehalten.

Aus dem Dokument DE 10 2016 223 478 A1 ist ein Verfahren zum Ermitteln von MR Bilddaten in Abhängigkeit von physiologischen Signalen bekannt. In dem Verfahren werden erste Bilddaten durch den Magnetresonanztomographen erfasst, physiologische Signaldaten erfasst, die ersten Bilddaten mit ersten Metadaten und die physiologischen Signaldaten mit zweiten Metadaten versehen, wobei die Metadaten eine zeitliche Zuordnung zu dem Zeitpunkt des Erfassens ermöglichen und die ersten Bilddaten und die physiologischen Signaldaten zu dem Auswerterechner übertragen.

Das Dokument US 2011/0118588 A1 beschreibt ein System mit einer Kombination aus Magnetresonanztomograph und Radiotherapiegerät mit einer Strahlenquelle. Ein Feldmagnet des Magnetresonanztomographen ist in der Lage, in weniger als 10 Minuten das Magnetfeld auf- bzw. abzubauen.

Eine Steuerung baut das Magnetfeld auf unter 20% des Wertes für eine Bilderfassung erforderlichen Wertes ab, wenn die Strahlungsquelle für die Radiotherapie verwendet werden soll und baut anschließend das Magnetfeld auf den ursprünglichen Wert auf, wenn das Magnetresonanzsystem für eine Bilderfassung genutzt werden soll.

Aus dem Dokument US 2013/0053678 A1 ist ein Magnetresonanztomograph bekannt, bei dem eine Störung eines beigestellten Therapiesystems durch den Magnetresonanztomographen reduziert wird, indem Hochfrequenz-empfindliche Aktionen des Therapiesystems nur ausgeführt werden, wenn eine Gradientenfeld-Aktivität des Magnetresonanztomographen unterdrückt ist.

Das Dokument US 2006/0279281 A1 beschreibt ein industrielles Qualitäts- und Prozessüberwachungssystem, das unter anderem eine dichte Matrix aus MRI/NMR-Systemen mit im Wesentlichen nicht überlappenden Magnetfeldern aufweist und ausgelegt ist, benachbarte Produktionslinien von Waren gleichzeitig zu überwachen.

Aus dem Dokument US 2016/0231403 A1 ist ein Verfahren zum Betrieb eines Magnetresonanztomographen bekannt. Die Anwesenheit eines benachbarten Magnetresonanzsystems wird erkannt. Das B0-Feld eines ersten Magnetresonanzsystems kann zur Veränderung der Larmorfrequenz so angepasst werden, dass sie von der Larmorfrequenz eines zweiten Magnetresonanzfrequenz in der Nähe abweicht.

Es ist eine Aufgabe der vorliegenden Erfindung, einen Magnetresonanztomographen, ein System aus Magnetresonanztomographen sowie ein Verfahren zum Betrieb bereitzustellen, das die oben genannten Störungen reduziert.

Die Aufgabe wird durch einen erfindungsgemäßen Magnetresonanztomographen nach Anspruch 1 oder 2 und ein erfindungsgemäßes System nach Anspruch 3 sowie ein Verfahren nach Anspruch 4 gelöst.

Der erfindungsgemäße Magnetresonanztomograph gemäss Anspruch 1 weist eine Steuereinheit zum Steuern der Bilderfassung auf, und eine Schnittstelle in Signalverbindung mit der Steuereinheit, wobei die Steuereinheit ausgelegt ist, eine Bilderfassung in Abhängigkeit von einem über die Schnittstelle von einem zweiten Magnetresonanztomographen empfangenen Signal zu synchronisieren, wobei die Schnittstelle zum Datenaustausch ausgelegt ist, wobei die Steuereinheit ausgelegt ist, mittels Informationsaustausches über die Schnittstelle eine Bilderfassung mit einem zweiten Magnetresonantomographen zu synchronisieren, wobei das Signal eine Information zu einem Zeitpunkt und einer Frequenz eines Sendevorgangs aufweist. Die Steuereinheit ist ausgelegt, einen Frequenzbereich eines Bilderfassungsvorganges in Abhängigkeit von der empfangenen Information zu ändern, wobei die Bilderfassung unterschiedliche Schichten umfasst, wobei diese durch ein Gradienten-Magnetfeld in z-Richtung und damit in der effektiven Larmorfrequenz des Magnetresonzsignals differenziert werden, wobei die Reihenfolge der Schichten bei der Bilderfassung so geordnet wird, dass nie gleichzeitig in beiden Magnetresonanztomographen eine Erfassung im gleichen Frequenzbereich erfolgt.

Bei einem erfindungsgemäßen Magnetresonanztomographen kann die Steuereinheit auch ausgelegt sein, ein Signal mit einer Information über eine bevorstehende Bilderfassung an einen anderen Magnetresonanztomographen zu senden. Dieses Merkmal ergänzt vergleichbar einer Stecker-Steckdose-Kombination den vorhergehenden Magnetresonanztomographen, der ausgelegt ist, ein Signal von einem anderen Magnetresonanztomographen zu empfangen. Die Information betrifft vorzugsweise einen Zeitpunkt der Aussendung und/oder Frequenz eines Anregungspulses. Vorzugsweise ist ein erfindungsgemäßer Magnetresonanztomograph, wie nachfolgend zu den abhängigen Ansprüchen erläutert, sowohl zum Senden als auch zum Empfangen eines Signals ausgelegt. Der Magnetresonanztomograph wird nachfolgend auch als erster Magnetresonanztomograph bezeichnet.

Die Steuereinheit des Magnetresonanztomographen ist dabei ausgelegt, die Frequenz eines Bilderfassungsvorganges in Abhängigkeit von der empfangenen Information zu ändern. Eine Bilderfassung umfasst unterschiedliche Schichten, wobei diese durch einen Gradienten-Magnetfeld in z-Achse und damit in der effektiven Larmorfrequenz des Magnetresonanzsignals differenziert werden. Es ist so ein gleichzeitiger Betrieb mit reduzierter Wechselwirkung möglich, sofern die Reihenfolge der Schichten bei der Bilderfassung so geordnet wird, dass nie gleichzeitig in beiden Systemen eine Erfassung im gleichen Frequenzbereich erfolgt.

Ein erfindungsgemäßes System weist einen ersten Magnetresonanztomographen und einen zweiten Magnetresonanztomographen auf. Der erste Magnetresonanztomograph und der zweite Magnetresonanztomograph weisen jeweils eine Schnittstelle und eine Steuereinheit auf. Der erste Magnetresonanztomograph und der zweite Magnetresonanztomograph stehen über die Schnittstellen in Signalverbindung. Die Signalverbindung ermöglicht zumindest die Übertragung eines Signals von dem ersten Magnetresonanztomographen zu dem zweiten Magnetresonanztomographen, es ist aber auch ein bidirektionaler Informationsaustausch denkbar. Denkbar sind Punkt-zu-Punktverbindungen auf elektrischem, optischem oder drahtlosem Weg. Möglich sind als Signalverbindung auch Netzwerke, wie LAN, WAN, im speziellen TCP/IP. Die Steuereinheit des ersten Magnetresonanztomographen ist ausgelegt, eine Information zu einem bevorstehenden Bilderfassungsvorgang über die Schnittstelle an den zweiten Magnetresonanztomographen zu senden. Denkbar ist ein Zeitpunkt einer geplanten Aussendung eines Anregungspulses in absoluter Zeit oder relativ zu dem Signal, oder auch zur Frequenz bzw. einem Frequenzbereich des Anregungspulses. Die Steuereinheit des zweiten Magnetresonanztomographen ist dabei ausgelegt, die Information über die Schnittstelle zu empfangen und eine Bilderfassung in Abhängigkeit von der empfangenen Information auszuführen. Erfindungsgemäss umfasst die Bilderfassung unterschiedliche Schichten, wobei diese durch ein Gradienten-Magnetfeld in z-Richtung und damit in der effektiven Larmorfrequenz des Magnetresonanzsignals differenziert werden, wobei die Reihenfolge der Schichten bei der Bilderfassung so geordnet wird, dass nie gleichzeitig in beiden Magnettomographen eine Erfassung im gleichen Frequenzbereich erfolgt.

Das erfindungsgemäße Verfahren ist zum Betrieb eines ersten Magnetresonanztomographen mit einer Steuereinheit zum Steuern der Bilderfassung und einer Schnittstelle in Signalverbindung mit der Steuereinheit vorgesehen. Das Verfahren weist den Schritt auf, ein Signal durch die Steuereinheit über die Schnittstelle von einem zweiten Magnetresonanztomographen zu empfangen. Dabei kann es sich um einen gezielten Informationsaustausch handeln, in dem der erste Magnetresonanztomograph über eine Datenschnittstelle eine Information bzw. Nachricht von dem zweiten Magnetresonanztomographen erhält, die Parameter wie Zeitpunkt und/oder Frequenz einer beabsichtigten Bilderfassung aufweist.

In einem anderen Schritt stellt die Steuereinheit des ersten Magnetresonanztomographen einen Parameter der Bilderfassung in Abhängigkeit von dem empfangenen Signal ein.

In einem möglichen Schritt ermittelt die Steuerung aus den empfangenen Magnetresonanzsignalen ein Bild und gibt es auf einem Display aus.

Erfindungsgemäss ändert die Steuereinheit des Magnetresonanztomographen die Frequenz eines Bilderfassungsvorganges in Abhängigkeit von der empfangenen Information. Eine Bilderfassung umfasst unterschiedliche Schichten, wobei diese durch einen Gradienten-Magnetfeld in z-Achse und damit in der effektiven Larmorfrequenz des Magnetresonanzsignals differenziert werden. Es ist so ein gleichzeitiger Betrieb mit reduzierter Wechselwirkung möglich, sofern die Reihenfolge der Schichten bei der Bilderfassung so geordnet wird, dass nie gleichzeitig in beiden Systemen eine Erfassung im gleichen Frequenzbereich erfolgt.

Auf vorteilhafte Weise ermöglichen die erfindungsgemässen Magnetresonanztomographen, das System aus zwei Magnetresonaztomographen und das Verfahren zum Betrieb, wechselseitige Störungen von zwei Magnetresonanztomographen bei gleichzeitigem Betrieb zu reduzieren. Auf vorteilhafte Weise ermöglicht die Differenzierung über die Frequenz eine gleichzeitige Erfassung von Magnetresonanzsignalen und damit eine bessere Nutzung der Untersuchungszeit.

In einer möglichen Ausführungsform des erfindungsgemäßen Verfahrens zum Betrieb eines ersten Magnetresonanztomographen und eines zweiten Magnetresonanztomographen weist das Verfahren weiterhin den Schritt auf, eine Information über eine bevorstehende Bilderfassung des zweiten Magnetresonanztomographen durch eine Steuereinheit des zweiten Magnetresonanztomographen zu ermitteln und in einem weiteren Schritt ein Signal mit der Information an den ersten Magnetresonanztomographen zu senden.

Ein weiterer Aspekt der Erfindung betrifft einen Magnetresonanztomographen mit einer Steuereinheit zum Steuern der Bilderfassung und einer Schnittstelle in Signalverbindung mit der Steuereinheit, wobei die Steuereinheit ausgelegt ist, eine Bilderfassung in Abhängigkeit von einem über die Schnittstelle von einem zweiten Magnetresonanztomographen empfangenen Signal zu synchronisieren, wobei der Magnetresonanztomograph als Schnittstelle einen Empfänger zum Empfang eines Magnetresonanzsignals aufweist und ausgelegt ist, ausserhalb einer Bilderfassung einen Anregungspuls des zweiten Magnetresonanztomographen mit einem Empfänger zu erfassen und die Bilderfassung in Abhängigkeit von dem erfassten Anregungspuls auszuführen, wobei die Bilderfassung unterschiedliche Schichten umfasst, wobei diese durch ein Gradienten-Magnetfeld in z-Richtung und damit in der effektiven Larmorfrequenz des Magnetresonanzsignals differenziert werden, wobei die Reihenfolge der Schichten bei der Bilderfassung so geordnet wird, dass nie gleichzeitig in beiden Magnetresonanztomographen eine Erfassung im gleichen Frequenzbereich erfolgt.

In einer möglichen Ausführungsform des erfindungsgemäßen Verfahrens zum Betrieb eines ersten Magnetresonanztomographen und eines zweiten Magnetresonanztomographen weist das Verfahren weiterhin den Schritt auf, eine Information über eine bevorstehende Bilderfassung des zweiten Magnetresonanztomographen durch eine Steuereinheit des zweiten Magnetresonanztomographen zu ermitteln und in einem weiteren Schritt ein Signal mit der Information an den ersten Magnetresonanztomographen zu senden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Systems aus Magnetresonanztomograph, Empfangsantenne und Anschlussleitung.
- Fig. 2: eine schematische Darstellung eines erfindungsgemäßen Systems aus mehreren Magnetresonanztomographen;
- Fig. 3: eine schematische Darstellung eines Ablaufplans einer Ausführungsform des erfindungsgemäßen Verfahrens.

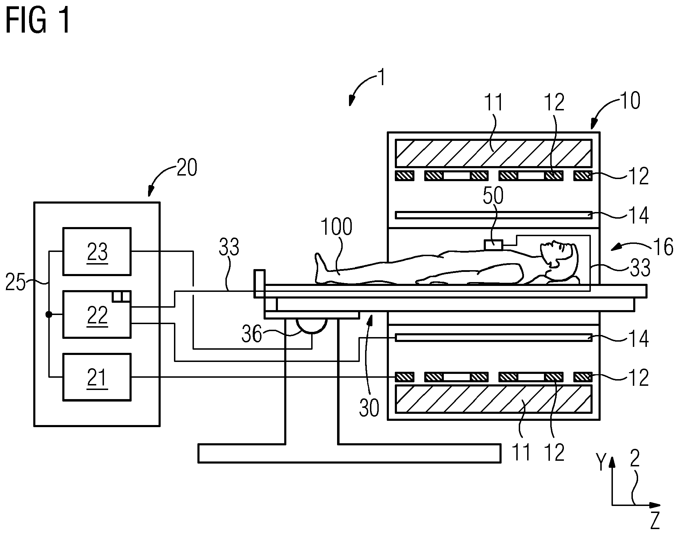

Fig. 1 zeigt eine schematische Darstellung einer Ausführungsform eines Magnetresonanztomographen 1 mit einer erfindungsgemäßen Lokalspule 50.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus. So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungspulse können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22. Die Steuereinheit 20 des Magnetresonanztomographen ist zumindest in einer Ausführungsform mit einer LAN-Schnittstelle 26 versehen, über die eine Kommunikation mit anderen Rechnern und insbesondere einem anderen, zweiten Magnetresonanztomographen 101 erfolgen kann.

Auf dem Patienten 100 ist eine Lokalspule 50 angeordnet, die über eine Anschlussleitung 33 mit der Hochfrequenzeinheit 22 und deren Empfänger verbunden ist.

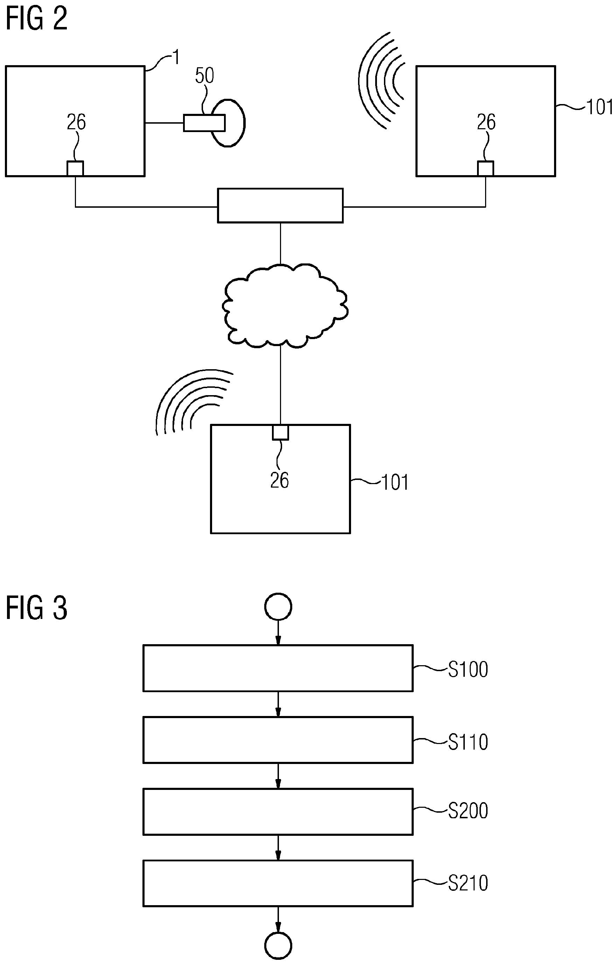

In Fig.2 ist ein erfindungsgemäßes System aus mehreren Magnetresonanztomographen 1 dargestellt. Dabei empfängt die Steuereinheit 20 eines Magnetresonanztomographen 1 über eine Schnittstelle ein Signal von einem zweiten Magnetresonanztomographen 101. Die Steuereinheit 21 ist dabei ausgelegt, eine Bilderfassung in Abhängigkeit von einem über die Schnittstelle von einem zweiten Magnetresonanztomographen 101 empfangenen Signal zu synchronisieren.

In der Fig. 2 sind mehrere mögliche Ausführungsformen gleichzeitig dargestellt. Die Schnittstelle kann zum einen die LAN-Schnittstelle 26 sein, über die der Magnetresonanztomograph 1 mit einem zweiten Magnetresonanztomographen 101 in Signalverbindung steht. Denkbar sind aber auch alle anderen Schnittstellen zum Informationsaustausch wie WIFI, WAN oder serielle oder parallele Punkt-zu-Punkt-Verbindungen.

Dabei ist es in einer Ausführungsform denkbar, dass der andere Magnetresonanztomograph 101 mit dem Signal eine Nachricht über eine geplante Bilderfassung sendet. Die Nachricht kann beispielsweise angeben, dass zu einem bestimmten Zeitpunkt t auf der Frequenz f eine Anregungspuls der Dauer d von dem anderen Magnetresonanztomograph 101 gesendet werden wird. Die Steuereinheit 20 des ersten Magnetresonanztomographen 1 synchronisiert dann die eigene Bilderfassung in Abhängigkeit von dieser Information.

Eine Möglichkeit ist, dass die Steuereinheit 20 einen eigenen Anregungspuls derart synchronisiert, dass er zur gleichen Zeit stattfindet, denn aufgrund der extrem hohen Feldstärken, die zur Anregung erforderlich sind, stören sich die Anregungspulse benachbarter zweiten Magnetresonanztomographen 101 aufgrund der bereits durch die Konstruktion der Magnetresonanztomographen 1, 101 gegebenen Dämpfung nicht.

Empfindlicher gegenüber Störungen ist hingegen der Empfang von Magnetresonanzsignalen aus dem Untersuchungsvolumen bzw. dem Patienten 100. Da hier eine Dämpfung gegenüber dem Anregungspuls von über 100 dB vorliegt, kann ein Anregungspuls eines benachbarten zweiten Magnetresonanztomographen 101 den Empfang eines MR-Signals auch bei vorhandener Abschirmung stören. Die Steuereinheit 20 des ersten Magnetresonanztomographen 1 kann deshalb die Bilderfassung derart planen und ausführen, dass diese nicht mit dem Anregungspuls des zweiten Magnetresonanztomographen 101 zusammenfällt. Beispielsweise können eigene Anregungspulse und die davon abhängigen Auslesesequenzen so gelegt werden, dass die Empfangszeitfenster des ersten Magnetresonanztomographen 1 nicht mit den Anregungspulsen des zweiten Magnetresonanztomographen 101 zusammenfallen.

Es ist dabei auch umgekehrt möglich, dass der zweite Magnetresonanztomograph 101 eine Information über einen geplanten Empfang sendet. Die Nachricht kann beispielsweise angeben, dass zu einem bestimmten Zeitpunkt t auf der Frequenz f für die Dauer d von dem zweiten Magnetresonanztomograph 101 ein Magnetresonanz-Signal aufgezeichnet werden soll. Der erste Magnetresonanztomograph 1 kann dann einen eigenen Sendevorgang so einstellen, dass in dem in der Nachricht angegebenen Zeitfenster kein Sendevorgang stattfindet, zumindest nicht auf einem Frequenzband, das die Frequenz f einschließlich einer in der Nachricht angegebenen Bandbreite umfasst.

Denkbar sind schließlich darüber hinaus kombinierte Nachrichten, in denen wechselseitig zwischen dem ersten Magnetresonanztomographen 1 und dem zweiten Magnetresonanztomographen 101 Sende- und Empfangsvorgänge abgestimmt werden, vorzugsweise derart, dass durch Verschachtelung die Bilderfassungsgeräte mit möglichst geringer Verzögerung ausgeführt werden können.

In einer anderen Ausführungsform, die in Fig. 2 dargestellt ist, ist es aber auch denkbar, dass das Signal eine Radiowelle eines Anregungspulses selbst ist und die Schnittstelle beispielsweise die Lokalspule 50 mit der Hochfrequenzeinheit 22. Vorzugsweise erfolgt dabei ein Empfang auch oder gerade in Zeiten, in denen der erste Magnetresonanztomograph 1 selbst kein MR-Signal aufzeichnet. Anhand des Anregungspulses kann die Steuereinheit 20 erfassen, dass ein zweiter Magnetresonanztomograph 101 gerade einen Anregungspuls gesendet hat und daher im Anschluss eine Erfassung eines Magnetresonanzsignals plant. Es ist dann beispielsweise denkbar, dass der erste Magnetresonanztomograph 1 dann für eine gewisse Zeit selbst keine Anregungspulse aussendet. Möglich wäre es auch, dass der erste Magnetresonanztomograph 1 den Anregungspuls des zweiten Magnetresonanztomographen selbst als Trigger-Puls nutzt und nahezu synchron einen eigenen Anregungspuls aussendet, da üblicherweise zwischen Anregungspuls und Empfang des Magnetresonanzsignals Pausen ohne Empfang und damit mögliche wechselseitige Störung liegen.

Unabhängig davon, ob das Aussenden eines Anregungspulses direkt über das empfangene elektromagnetische Feld des Pulses oder über eine Nachricht über die Datenschnittstelle erkannt wird, ist es dabei auch denkbar, dass die Steuereinheit 20 die Frequenz des nächsten Anregungspulses in Abhängigkeit von dem Signal ändert. Bei der Magnetresonanztomographie werden einzelne Schichten entlang der Richtung des B0-Feldes, üblicherweise entlang der z-Achse 2, durch ein überlagertes Gradientenfeld in z-Richtung in der Frequenz differenziert und damit unterscheidbar. Erfindungsgemäss ändert die Steuereinheit 20 die Reihenfolge der Abtastung einzelner Schichten, sodass der erste Magnetresonanztomograph 1 und der zweite Magnetresonanztomograph 101 jeweils Schichten mit unterschiedlicher Mittenfrequenz erfassen und so durch die unterschiedlichen Frequenzen ein Übersprechen bzw. eine Wechselwirkung vermieden werden. Ein zusätzlicher Freiheitsgrad, den die Steuereinheit 20 nutzen kann, ist dabei auch die Lage des Patienten 100 auf der verfahrbaren Patientenliege 30 relativ zu dem Isozentrum des Feldmagneten 10. Indem der Patient 100 um ein Stück entlang der der z-Achse verfahren wird, ändert sich durch die unterschiedliche Lage in Bezug auf das z-Gradientenfeld auch die Larmorfrequenz für eine Schicht. Der erste Magnetresonanztomograph 1 kann also durch eine Relativbewegung des Patienten entlang der z-Achse auch die gleiche Schicht im Körper des Patienten 100 mit unterschiedlichen Frequenzen erfassen, sodass eine Wechselwirkung mit dem zweiten Magnetresonanztomographen 101 vermieden werden kann.

In Fig. 3 ist ein schematischer Ablaufplan eines erfindungsgemäßen Verfahrens angegeben.

In einem Schritt S200 empfängt die Steuereinheit 20 ein Signal über die Schnittstelle von einem zweiten Magnetresonanztomographen 101. Dabei kann die Schnittstelle beispielsweise eine LAN-Schnittstelle 26, eine WAN-Schnittstelle oder auch eine Punkt-zu-Punkt Datenschnittstelle sein. Vorzugsweise ist die Schnittstelle dazu ausgelegt, Daten von dem anderen Magnetresonanztomographen 101 mit Information zu einem Bilderfassungsvorgang des anderen Magnetresonanztomographen 101 zu empfangen. Die Daten können einen Sendevorgang eines Anregungspulses und/oder eines Empfangsvorganges von MR-Signalen betreffen. Vorzugsweise umfassen die Daten Information zu Frequenz und Zeit, beispielsweise Mittenfrequenz, Bandbreite Startzeitpunkt und/oder Dauer.

Es ist in einer Ausführungsform aber auch denkbar, dass das Signal ein elektromagnetisches Feld des Anregungspulses selbst und die Schnittstelle eine Antenne wie die Lokalspule 50 und die Hochfrequenzeinheit 22 zum Empfang von MR-Signalen ist. Die Hochfrequenzeinheit 22 überwacht dabei vorzugsweise zwischen eigenen Anregungspulsen und MR-Signalerfassungen das Frequenzband, in dem der erste Magnetresonanztomograph 1 arbeitet. Mit Empfang eines Anregungspulses, der sich durch Frequenz, Dauer und/oder Verlauf auszeichnet, kann die Steuereinheit 20 eine Aktivität des zweiten Magnetresonanztomographen 101 erfassen.

In einem weiteren Schritt S210 stellt die Steuereinheit 20 einen Parameter der Bilderfassung in Abhängigkeit von dem empfangenen Signal ein. Beispielsweise kann die Steuereinheit 20 eine Sequenz einer eigenen Bilderfassung so verzögern, dass sie durch den Anregungspuls des zweiten Magnetresonanztomographen 101 nicht gestört wird. Auch ist es denkbar, einen eigenen Anregungspuls so zu verzögern, dass er eine Sequenz des zweiten Magnetresonanztomographen 101 nicht stört, beispielsweise indem der Anregungspuls gleichzeitig erfolgt oder auch so spät, dass er die Erfassung der Magnetresonanzsignale nicht stört. Auch ist durch Austausch von Daten zur Sequenz zwischen den beiden Magnetresonanztomographen 1; 101 eine Verschachtelung der Sequenzen derart möglich, dass ein Minimum an Verzögerung für beide Sequenzen erreicht wird.

Über eine zeitliche Variation hinaus ist auch die Frequenz ein denkbarer Parameter, der von der Steuereinheit 20 veränderbar ist. Bei einer Abtastung mehrere Schichten entlang der z-Achse wird die räumliche Differenzierung durch den Gradienten des Magnetfeldes entlang der z-Achse und damit resultierenden unterschiedlichen Larmorfrequenzen erreicht. Durch Ändern der Reihenfolge der zu erfassenden Schichten kann die gleichzeitige Nutzung der gleichen Frequenz beispielsweise vermieden werden. Selbst bei der unveränderter Schicht kann über eine Veränderung der Position der Patientenliege 30 entlang der z-Achse eine andere Frequenz gewählt werden und so eine Kollision vermieden werden.

Anschließend erfolgt durch den Magnetresonanztomographen 1, gesteuert durch die Steuereinheit 20 eine Bilderfassung gemäß dem bzw. den eingestellten Parametern. Abschließend kann das Bild auf einem Display ausgegeben werden.

In einer möglichen Ausführungsform des erfindungsgemäßen Verfahrens wird von der Steuereinheit 20 des zweiten Magnetresonanztomographen 101 in Schritt S100 eine Information über eine bevorstehende Bilderfassung des zweiten Magnetresonanztomographen 101 ermittelt. Die Steuereinheit 20 ermittelt beispielsweise eine oder mehrere Informationen aus der folgenden Aufzählung: Frequenz eines Anregungspulses, Frequenzverteilung eines Anregungspulses, Start-Zeitpunkt des Aussendens des Anregungspulses, Dauer des Anregungspulses, Frequenz einer bzw. mehrerer MR-Signalerfassungen, Frequenzverteilung der MR-Signalerfassung(en), Startzeitpunkt und Dauer der MR-Signalerfassung(en).

In einem weiteren Schritt S110 sendet der zweite Magnetresonanztomograph 101 ein Signal mit der Information an den ersten Magnetresonanztomographen 1. Dies kann beispielsweise über die LAN-Schnittstellen 26 und ein damit verbundenes Datennetzwerk erfolgen.

Das erfindungsgemäße Verfahren kann dabei noch um weitere Schritte ergänzt sein, in denen die Abstimmung der Bilderfassung zwischen den beiden Magnetresonanztomographen im Detail abgestimmt wird. Beispielsweise kann die Nutzung unterschiedliche Frequenzen zu einem bestimmten vereinbart werden, indem die Reihenfolge der Abtastung von Schichten angepasst wird. Es ist auch denkbar, dass das Senden von Hochfrequenzpulsen so eingestellt wird, dass beide Magnetresonanztomographen 1; 101 gleichzeitig senden, da Störungen vor allem während des Empfangs von MR-Signalen in dem ersten Magnetresonanztomographen 1 auftreten, wenn der zweite Magnetresonanztomograph 101 sendet. Darüber hinaus sind weitere Koordinierungen von Sende- und Empfangstätigkeiten möglich, beispielsweise durch Empfangspausen, die durch Gradientenumschaltungen entstehen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanztomograph mit einer Steuereinheit (20) zum Steuern der Bilderfassung und einer Schnittstelle in Signalverbindung mit der Steuereinheit (20), wobei die Steuereinheit (20) ausgelegt ist, eine Bilderfassung in Abhängigkeit von einem über die Schnittstelle von einem zweiten Magnetresonanztomographen (101) empfangenen Signal zu synchronisieren,
wobei die Schnittstelle zum Datenaustausch ausgelegt ist, und wobei die Steuereinheit (20) ausgelegt ist, mittels Informationsaustausches über die Schnittstelle eine Bilderfassung mit einem zweiten Magnetresonanztomographen (101) zu synchronisieren,
wobei das Signal eine Information zu einem Zeitpunkt und einer Frequenz eines Sendevorganges aufweist,
**dadurch gekennzeichnet, dass**
die Steuereinheit (20) ausgelegt ist, einen Frequenzbereich eines Bilderfassungsvorganges, in Abhängigkeit von der empfangenen Information zu ändern,
wobei die Bilderfassung unterschiedliche Schichten umfasst,
wobei diese durch ein Gradienten-Magnetfeld in z-Richtung und damit in der effektiven Larmorfrequenz eines Magnetresonanzsignals differenziert werden, wobei die Reihenfolge der Schichten bei der Bilderfassung so geordnet wird, dass nie gleichzeitig in beiden Magnetresonanztomographen eine Erfassung im gleichen Frequenzbereich erfolgt.

2. Magnetresonanztomograph mit einer Steuereinheit (20) zum Steuern der Bilderfassung und einer Schnittstelle in Signalverbindung mit der Steuereinheit (20), wobei die Steuereinheit (20) ausgelegt ist, eine Bilderfassung in Abhängigkeit von einem über die Schnittstelle von einem zweiten Magnetresonanztomographen (101) empfangenen Signal zu synchronisieren,
**dadurch gekennzeichnet, dass**
der Magnetresonanztomograph (1) als Schnittstelle einen Empfänger zum Empfang eines Magnetresonanzsignals aufweist und ausgelegt ist, außerhalb einer Bilderfassung einen Anregungspuls des zweiten Magnetresonanztomographen (101) mit dem Empfänger zu erfassen und die Bilderfassung in Abhängigkeit von dem erfassten Anregungspuls auszuführen, wobei die Bilderfassung unterschiedliche Schichten umfasst, wobei diese durch ein Gradienten-Magnetfeld in z-Richtung und damit in der effektiven Larmorfrequenz differenziert werden, wobei die Reihenfolge der Schichten bei der Bilderfassung so geordnet wird, dass nie gleichzeitig in beiden Magnetresonanztomographen eine Erfassung im gleichen Frequenzbereich erfolgt.

3. System aus einem ersten Magnetresonanztomographen (1) und einem zweiten Magnetresonanztomographen (101), wobei der erste Magnetresonanztomograph (1) und der zweite Magnetresonanztomograph (101) jeweils eine Schnittstelle und eine Steuereinheit (20) aufweisen und der erste Magnetresonanztomograph (1) mit dem zweiten Magnetresonanztomographen (101) über die Schnittstellen in Signalverbindung steht, wobei die Steuereinheit (20) des ersten Magnetresonanztomographen (1) ausgelegt ist, eine Information zu einem bevorstehenden Bilderfassungsvorgang über die Schnittstelle an den zweiten Magnetresonanztomographen (101) zu senden und die Steuereinheit (20) des zweiten Magnetresonanztomographen (1) ausgelegt ist, die Information über die Schnittstelle zu empfangen und eine Bilderfassung in Abhängigkeit von der empfangenen Information auszuführen,
**dadurch gekennzeichnet, dass** die Bilderfassung unterschiedliche Schichten umfasst, wobei diese durch ein Gradienten-Magnetfeld in z-Richtung und damit in der effektiven Larmorfrequenz eines Magnetresonanzsignals differenziert werden, wobei die Reihenfolge der Schichten bei der Bilderfassung so geordnet wird, dass nie gleichzeitig in beiden Magnetresonanztomographen eine Erfassung im gleichen Frequenzbereich erfolgt.

4. Verfahren zum Betrieb eines ersten Magnetresonanztomographen (1) mit einer Steuereinheit (20) zum Steuern der Bilderfassung und einer Schnittstelle in Signalverbindung mit der Steuereinheit, wobei das Verfahren die Schritte aufweist:
(S200) Empfangen eines Signals durch die Steuereinheit (20) über die Schnittstelle von einem zweiten Magnetresonanztomographen (101);
(S210) Einstellen eines Parameters der Bilderfassung in Abhängigkeit von dem empfangenen Signal durch die Steuereinheit (20) ;
(S220) Ausführen einer Bilderfassung gemäß dem eingestellten Parameter,
**dadurch gekennzeichnet, dass** die Bilderfassung unterschiedliche Schichten umfasst, wobei diese durch ein Gradienten-Magnetfeld in z-Achse und damit in der effektiven Larmorfrequenz eines Magnetresonanzsignals differenziert werden, wobei als Parameter der Bilderfassung die Reihenfolge der Schichten bei der Bilderfassung so geordnet wird, dass nie gleichzeitig in beiden Magnetresonanztomographen eine Erfassung im gleichen Frequenzbereich erfolgt.

5. Verfahren zum Betrieb eines ersten Magnetresonanztomographen (1) nach Anspruch 4 mit einem zweiten Magnetresonanztomographen (101), wobei das Verfahren weiterhin den Schritt aufweist:
(S100) Ermitteln einer Information über eine bevorstehende Bilderfassung des zweiten Magnetresonanztomographen (101) durch eine Steuereinheit (20) des zweiten Magnetresonanztomographen (101);
(S110) Senden eines Signals mit der Information an den ersten Magnetresonanztomographen (1).

6. Computerprogrammprodukt, welches direkt in einen Prozessor einer Steuereinheit (20) des ersten Magnetresonanztomographen (1) und ggf. zweiten Magnetresonanztomographen (101) ladbar ist, mit Programmcode-Mitteln, um alle Schritte eines Verfahrens nach einem der Ansprüche 4 oder 5 auszuführen, wenn das Programmprodukt auf der Steuereinheit (20) ausgeführt wird.

7. Computerlesbares Speichermedium mit darauf gespeicherten elektronisch lesbaren Steuerinformationen, welche derart ausgestaltet sind, dass sie bei Verwendung des Speichermediums in der Steuereinheit (20) des ersten Magnetresonanztomographen (1) und ggf. zweiten Magnetresonanztomographen (101) das Verfahren nach einem der Ansprüche 4 oder 5 durchführen.

## Claims

1. Magnetic resonance tomography scanner having a control unit (20) for controlling the image acquisition and an interface connected to the control unit (20) for signal communication purposes, wherein the control unit (20) is configured for synchronising an image acquisition as a function of a signal received via the interface from a second magnetic resonance tomography scanner (101),
wherein the interface is configured for exchanging data and wherein the control unit (20) is configured for synchronising an image acquisition with a second magnetic resonance tomography scanner (101) by means of information exchange via the interface,
wherein the signal includes information relating to a time and frequency of a transmit process,
**characterised in that**
the control unit (20) is configured for changing a frequency range of an image acquisition process as a function of the received information,
wherein the image acquisition includes different slices, wherein these are differentiated by a gradient magnetic field in the z-direction and thus in the effective Larmor frequency of a magnetic resonance signal, wherein the sequence of slices is ordered during the image acquisition so that an acquisition in the same frequency range never takes place simultaneously in both magnetic resonance tomography scanners.

2. Magnetic resonance tomography scanner having a control unit (20) for controlling the image acquisition and an interface connected to the control unit (20) for signal communication purposes, wherein the control unit (20) is configured for synchronising an image acquisition as a function of a signal received from a second magnetic resonance tomography scanner (101) via the interface,
**characterised in that**
the magnetic resonance tomography scanner (1) has as an interface a receiver for receiving a magnetic resonance signal and is configured for detecting an excitation pulse of the second magnetic resonance tomography scanner (101) outside of an image acquisition and for performing the image acquisition as a function of the detected excitation pulse, wherein the image acquisition includes different slices, wherein these are differentiated by a gradient magnetic field in the z-direction and thus in the effective Larmor frequency, wherein the sequence of slices is ordered during the image acquisition so that an acquisition in the same frequency range never takes place simultaneously in both magnetic resonance tomography scanners.

3. System composed of a first magnetic resonance tomography scanner (1) and a second magnetic resonance tomography scanner (101), wherein the first magnetic resonance tomography scanner (1) and the second magnetic resonance tomography scanner (101) each have an interface and a control unit (20) and the first magnetic resonance tomography scanner (1) is connected to the second magnetic resonance tomography scanner (101) via the interfaces for signal communication purposes, wherein the control unit (20) of the first magnetic resonance tomography scanner (1) is configured for sending information relating to an upcoming image acquisition process via the interface to the second magnetic resonance tomography scanner (101) and the control unit (20) of the second magnetic resonance tomography scanner (1) is configured for receiving the information via the interface and for performing an image acquisition as a function of the received information,
**characterised in that**
the image acquisition includes different slices, wherein these are differentiated by a gradient magnetic field in the z-direction and thus in the effective Larmor frequency of a magnetic resonance signal, wherein the sequence of the slices during the image acquisition is ordered so that an acquisition in the same frequency range never takes place simultaneously in both magnetic resonance tomography scanners.

4. Method for operating a first magnetic resonance tomography scanner (1) having a control unit (20) for controlling the image acquisition and an interface connected to the control unit for signal communication purposes, wherein the method has the steps of:
(S200) receiving a signal by the control unit (20) via the interface from a second magnetic resonance tomography scanner (101);
(S210) setting an image acquisition parameter as a function of the received signal by the control unit (20);
(S220) performing an image acquisition in accordance with the set parameter,
**characterised in that** the image acquisition includes different slices, wherein these are differentiated by a gradient magnetic field in the z-axis and thus in the effective Larmor frequency of a magnetic resonance signal, wherein as a parameter of the image acquisition the sequence of the slices is ordered during the image acquisition so that an acquisition in the same frequency range never takes place simultaneously in both magnetic resonance tomography scanners.

5. Method for operating a first magnetic resonance tomography scanner (1) according to claim 4 having a second magnetic resonance tomography scanner (101), wherein the method further comprises the step of:
(S100) ascertaining information about an upcoming image acquisition of the second magnetic resonance tomography scanner (101) by a control unit (20) of the second magnetic resonance tomography scanner (101);
(S110) sending a signal containing the information to the first magnetic resonance tomography scanner (1).

6. Computer program product which can be loaded directly into a processor of a control unit (20) of the first magnetic resonance tomography scanner (1) and possibly second magnetic resonance tomography scanner (101) and which has program code means for performing all steps of a method according to one of claims 4 or 5 when the program product is executed on the control unit (20).

7. Computer-readable storage medium on which is stored electronically readable control information which is embodied in such a way that it performs the method according to one of claims 4 or 5 when the storage medium is used in the control unit (20) of the first magnetic resonance tomography scanner (1) and possibly second magnetic resonance tomography scanner (101).

## Revendications

1. Tomodensitomètre à résonnance magnétique comprenant une unité (20) de commande pour la commande de la prise d'image et une interface en communication de signal avec l'unité (20) de commande, dans lequel l'unité (20) de commande est conçue pour synchroniser une prise d'image en fonction d'un signal reçu par un deuxième tomodensitomètre (101) à résonnance magnétique par l'intermédiaire de l'interface,
dans lequel l'interface est conçue pour l'échange de données, et dans lequel l'unité (20) de commande est conçue pour synchroniser, au moyen d'un échange d'information par l'interface, une prise d'image avec un deuxième tomodensitomètre (101) à résonnance magnétique, dans lequel le signal a une information à un instant et à une fréquence d'une opération d'envoi,
**caractérisé en ce que** l'unité (20) de commande est conçue pour modifier un domaine de fréquence d'une opération de prise d'image en fonction de l'information reçue,
dans lequel la prise d'image comprend des couches différentes, dans lequel celles-ci sont différenciées par un champ magnétique de gradient dans une direction z et ainsi à la fréquence de Larmor effective d'un signal de résonnance magnétique, dans lequel la succession des couches est ordonnée lors de la prise d'image, de manière à ne jamais produire en même temps, dans les deux tomodensitomètres à résonnance magnétique, une prise dans le même domaine de fréquence.

2. Tomodensitomètre à résonnance magnétique comprenant une unité (20) de commande pour la commande de la prise d'image et une interface en communication de signal avec l'unité (20) de commande, dans lequel l'unité (20) de commande est conçue pour synchroniser une prise d'image en fonction d'un signal reçu d'un deuxième tomodensitomètre (101) à résonnance magnétique par l'intermédiaire de l'interface,
**caractérisé en ce que**
le tomodensitomètre (1) à résonnance magnétique a comme interface un récepteur de réception d'un signal de résonnance magnétique et est conçu pour détecter par le récepteur, en dehors d'une prise d'image, une impulsion d'excitation du deuxième tomodensitomètre (101) à résonnance magnétique et pour effectuer la prise d'image en fonction de l'impulsion d'excitation détectée, dans lequel la prise d'image comprend des couches différentes, dans lequel celles-ci sont différenciées par un champ magnétique de gradient dans la direction z et ainsi à la fréquence de Larmor effective, dans lequel la succession des couches est ordonnée dans la prise d'image, de manière à ne produire jamais en même temps, dans les deux tomodensitomètres à résonnance magnétique, une prise dans le même domaine de fréquence.

3. Système composé d'un premier tomodensitomètre (1) à résonnance magnétique et d'un deuxième tomodensitomètre (101) à résonnance magnétique, dans lequel le premier tomodensitomètre (1) à résonnance magnétique et le deuxième tomodensitomètre (101) à résonnance magnétique ont chacun une interface et une unité (20) de commande, et le premier densitomètre (1) à résonnance magnétique est en communication de signal avec le deuxième tomodensitomètre (101) à résonnance magnétique par l'intermédiaire des interfaces, dans lequel l'unité (20) de commande du premier densitomètre (1) à résonnance magnétique est conçue pour envoyer de l'information sur une opération de prise d'image imminente au deuxième tomodensitomètre (101) à résonnance magnétique par l'intermédiaire de l'interface et l'unité (20) de commande du deuxième tomodensitomètre (1) à résonnance magnétique est conçue pour recevoir l'information par l'intermédiaire de l'interface et pour effectuer la prise d'image en fonction de l'information reçue, **caractérisé en ce que** la prise d'image comprend des couches différentes, dans lequel celles-ci sont différenciées par un champ magnétique de gradient dans une direction z et ainsi à la fréquence de Larmor effective d'un signal de résonnance magnétique, dans lequel la succession des couches lors de la prise d'image est ordonnée, de manière à ce qu'une prise dans le même domaine de fréquence ne se produise jamais en même temps dans les deux tomodensitomètres à résonnance magnétique.

4. Procédé pour faire fonctionner un premier tomodensitomètre (1) à résonnance magnétique ayant une unité (20) de commande pour la commande de la prise d'image et une interface en communication de signal avec l'unité de commande, dans lequel le procédé comprenant les stades :
(S200) réception d'un signal par l'unité (20) de commande d'un deuxième tomodensitomètre (101) à résonnance magnétique par l'intermédiaire de l'interface ;
(S210) réglage d'un paramètre de la prise d'image en fonction du signal reçu par l'unité (20) de commande ;
(S220) réalisation de la prise d'image suivant le paramètre réglé, **caractérisé en ce que** la prise d'image comprend des couches différentes, dans lequel celles-ci sont différenciées par un champ magnétique de gradient dans une direction z et ainsi à la fréquence de Larmor efficace d'un signal de résonnance magnétique, dans lequel, comme paramètre de la prise d'image, on ordonne la succession des couches lors de la prise d'image, de manière à ce qu'une prise dans le même domaine de fréquence ne se produise jamais en même temps dans les deux tomodensitomètres à résonnance magnétique.

5. Procédé pour faire fonctionner un premier tomodensitomètre (1) à résonnance magnétique suivant la revendication 4 par un deuxième tomodensitomètre (101) à résonnance magnétique, dans lequel le procédé a en outre le stade :
(S100) détermination d'une information sur une prise d'image imminente du deuxième tomodensitomètre (101) à résonnance magnétique par une unité (20) de commande du deuxième tomodensitomètre (101) à résonnance magnétique ;
(S110) envoi d'un signal ayant l'information au premier tomodensitomètre (1) à résonnance magnétique.

6. Produit de programme d'ordinateur, qui peut être chargé directement dans un processeur d'une unité (20) de commande du premier tomodensitomètre (1) à résonnance magnétique, et le cas échéant du deuxième tomodensitomètre (101) à résonnance magnétique, ayant des moyens de code de programme, pour effectuer tous les stades d'un procédé suivant l'une des revendications 4 ou 5, lorsque le produit de programme est réalisé sur l'unité (20) de commande.

7. Support de mémoire, déchiffrable par ordinateur, sur lequel sont mises en mémoire des informations de commande déchiffrables électroniquement, qui sont conformées de manière à ce qu'elles effectuent, lors de l'utilisation du support de mémoire dans l'unité (20) de commande du premier tomodensitomètre (1) à résonnance magnétique, et le cas échéant du deuxième tomodensitomètre (101) à résonnance magnétique, le procédé suivant l'une des revendications 4 ou 5.
